# EUROPÄISCHE PATENTSCHRIFT

(11) **EP 0 634 139 B1**
(45) Veröffentlichungstag und Bekanntmachung des Hinweises auf die Patenterteilung: **04.03.1998**
(21) Anmeldenummer: 94107721.6
(22) Anmeldetag: 18.05.1994
(51) Int. Cl.: A61B 17/00

(54) **Zerlegbares medizinisches Instrument**
Dismountable medical instrument
Instrument médical démontable

(30) Priorität: 14.07.1993 DE 4323584
(43) Veröffentlichungstag der Anmeldung: 18.01.1995
(73) Patentinhaber: DELMA ELEKTRO-UND MEDIZINISCHE APPARATEBAU GESELLSCHAFT mbH, D-78532 Tuttlingen (DE)
(72) Erfinder: Fritzsch, Gernod, Dr, Ing., D-78532 Tuttlingen (DE); Hermle, Michael, Dipl.-Ing. (FH), D-78532 Tuttlingen (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(56) Entgegenhaltungen:
- US-A- 4 493 319

## Beschreibung

Die Erfindung betrifft ein zerlegbares medizinisches Instrument, insbesondere Hochfrequenz-Chirurgieinstrument nach dem Oberbegriff des Patentanspruchs 1.

Bei medizinischen Instrumenten, insbesondere chirurgischen Hochfrequenz-Chirurgieinstrumenten für die laparoskopische oder minimal-invasive Chirurgie besteht wegen des immer komplexer werdenden Instrumentariums in verstärktem Maße das Problem der Reinigung. Um dieses Problem zu lösen, sind bereits mehr oder weniger weit zerlegbare Instrumente bekannt. Häufig ist gleichwohl eine optimale und vollständige Reinigung problematisch. Bei Instrumenten mit beweglichem Arbeitsteil, d.h. insbesondere axial beweglicher Arbeitselektrode ist der Zusammenbau oft sehr schwierig und von ungeübtem Klinikpersonal nicht durchzuführen.

Ein zerlegbares medizinisches Instrument der eingangs genannten Art ist aus der US-A-4 493 319 bekannt. Dieses Instrument, mit dem beispielsweise zum Zwecke der Sterilisation elastische Ringe zum Abschnüren der Eileiter einer Patientin in deren Körper eingebracht werden können, umfaßt einen zentralen Arbeitsstab, an dessen vorderem Ende zwei Greifelemente vorgesehen sind. Der zentrale Arbeitsstab ist in einem Führungsrohr verschiebbar gelagert, wobei durch Verschieben von Arbeitsstab und Führungsstab relativ zueinander die Greifelemente zwischen einer offenen Freigabestellung und einer geschlossenen Greifstellung verstellbar sind. Sowohl der Arbeitsstab als auch das Führungsrohr sind durch einen zweiteiligen Handgriff hindurchgeführt, so daß die hinteren Enden des Arbeitsstabs und des Führungsrohrs an der Rückseite des rückwärtigen Handgriffteils austreten. Der Arbeitsstab ist über eine durch den hinteren Handgriffteil verschiebbar geführte Schubstange an dem vorderen Handgriffteil befestigt, so daß durch Zurückziehen des vorderen Handgriffteils der Arbeitsstab zurückgezogen wird. Dadurch werden die Greifelemente in das Innere des Führungsrohrs hineingezogen und von der Freigabestellung in die Greifstellung zusammengedrückt. Nachteilig an dieser Vorrichtung ist zum einen der relativ komplizierte Aufbau, wobei insbesondere gewährleistet sein muß, daß die Schubstange zu dem Arbeitsstab bzw. dem Führungsrohr exakt parallel geführt ist, um ein Verklemmen beim Verschieben des vorderen Handgriffabschnitts zu vermeiden.

Der Erfindung liegt die Aufgabe zugrunde, ein medizinisches Instrument, insbesondere Hochfrequenz-Chirurgieinstrument der eingangs genannten Gattung zu schaffen, welches in optimaler Weise zu reinigen und auch von ungeübtem Klinikpersonal zu zerlegen und wieder zusammenzusetzen ist.

Zur Lösung dieser Aufgabe sind die Merkmale des kennzeichnenden Teils des Anspruches 1 vorgesehen. Vorteilhafte Weiterbildungen der Erfindung entnimmt man den Unteransprüchen.

Bei einem bevorzugten erfindungsgemäßen Ausführungsbeispiel sind sämtliche, vorzugsweise koaxial zueinander angeordnete Teile, nämlich der Arbeitsstab, das Führungsrohr, gegebenenfalls das Außenrohr und der rohrartig ausgebildete Handgriff axial voneinander trennbar ausgebildet, wobei die im Betrieb vorhandene lösbare Verbindung durch einfache Rast- und/oder Riegel- und/oder Schnappverbindungen gewährleistet ist.

Von besonderem Vorteil ist die Ausführungsform nach Anspruch 9, weil hierbei der gleiche axiale Verschiebemechanismus für die im Betrieb vorgesehene Relativverschiebung zwischen Arbeitsstab und Führungsrohr und für die gegebenenfalls gewünschte Lösung zwischen Führungsrohr und Führungshülse ausgenutzt wird. Die axiale Verschiebung des Führungsrohres kann z.B. dem Zweck dienen, zwei Koagulationselektroden am distalen Ende des Instrumentes wahlweise einander zu nähern oder voneinander zu entfernen, um damit beim Koagulieren menschliches Gewebe ergreifen zu können.

Die Erfindung wird im folgenden beispielsweise anhand der Zeichnung beschrieben; in dieser zeigt:
- Fig. 1: eine teilweise geschnittene Seitenansicht eines erfindungsgemäßen Hochfrequenz-Chirurgieinstrumentes und
- Fig. 2: einen Schnitt nach Linie II-II in Fig. 1.

Nach der Zeichnung erstreckt sich ein an seinem distalen Ende zwei federnd voneinander weg vorgespannte Arbeitselektroden 25 aufweisendes, einen zentralen Arbeitsstab bildendes Elektrodenrohr 11 koaxial und durch ein es umgebendes Führungsrohr 12, welches an seinem hinteren Ende über den Umfang gleichmäßig verteilt axial verlaufende Rastzungen 24 trägt, die sich axial durch eine zentrale Bohrung 31 in einer Führungshülse 20 erstrecken, welche axial verschiebbar in einem rohrartigen Betätigungshandgriff 14 angeordnet ist. Das Elektrodenrohr 11 verlauft durch die Führungshülse 20 hindurch, und zwar radial innerhalb der Rastzungen 24 und derart, daß es das Außer-Eingriff-Kommen von Rastzungen 24 und Führungshülse 20 verhindert. Der hintere Endbereich des Elektrodenrohres 11 erstreckt sich durch eine Bohrung 32 in einem an einer handgriffesten Ringwand 38 vorgesehenen Kulissenansatz 33 bis zu einem im Handgriff 14 querverschiebbar angeordneten Kupplungsriegel 16, der von einer Riegelfeder 21 diametral gegen einen Druckknopf 15 vorgespannt ist, welcher sich durch eine Bohrung 34 in der Wand des Handgriffes 14 radial nach außen erstreckt und dessen Bewegung radial nach außen durch eine im Innern des Handgriffs 14 liegende radiale Erweiterung 35 begrenzt ist.

Der Kupplungsriegel 16 ist durch nur schematisch angedeutete Querführungen 36 derart in Richtung quer zur Achse 47 des Instrumentes geführt, daß er axiale Belastungen aufnehmen kann. Der Kupplungsriegel 16 weist eine zentrale Öffnung 37 auf, die den hinteren Endbereich des Elektrodenrohres 11 umgibt und deren untere Berandung durch die Riegelfeder 21 in eine im Elektrodenrohr 11 vorgesehene Ringnut 26 eingreift. Da der plattenförmig ausgebildete Kupplungsriegel 16 und die Ringnut 26 komplementär zueinander ausgebildet sind, liegt somit ein axial formschlüssiger Eingriff zwischen dem Kupplungsriegel 16 und dem Elektrodenrohr 11 vor, welcher eine axiale Verschiebung des Elektrodenrohrs 11 innerhalb des Handgriffes 14 und vorzugsweise auch die sonst mögliche Drehung des Elektrodenrohres 11 um die Achse 47 verhindert.

Durch Drücken auf den Druckknopf 15 in Richtung des Pfeiles f kann die formschlüssige Verbindung zwischen dem Kupplungsriegel 16 und dem hinteren Endbereich des Elektrodenrohrs 11 gelöst werden, wodurch das Elektrodenrohr 11 nach vorn aus dem Führungsrohr 12 herausgezogen werden kann.

Die Führungshülse 20 ist durch eine sich an einer Ringwand 38 abstützende Rückstellfeder 19 nach vorn vorgespannt. Ein radial von der Führungshülse 20 durch einen in Axialrichtung länglichen Radialschlitz 39 des Handgriffs 14 vorstehender Betätigungsbügel 18 wird durch die Rückstellfeder 19 gegen einen durch das vordere Ende des Radialschlitzes 39 gebildeten Anschlag 30 gedrückt, wodurch die Bewegung des Führungsrohrs 12 nach vorn begrenzt wird.

Die Rastzungen 24 gehen am vorderen Ende über eine Ringstufe 40 in das Führungsrohr 12 über, wodurch eine Verschiebung des Führungsrohres 12 relativ zur Führungshülse 20 nach hinten verhindert wird. Das hintere Ende der Führungshülse 20 wird von den Rastköpfen 24' der Rastzungen 24 derart hintergriffen, daß im eingerasteten Zustand auch eine Bewegung des Führungsrohrs 12 relativ zur Führungshülse 20 nach vorne verhindert ist.

In den Radialschlitz 39 greift von hinten ein um ein Quergelenk 41 hochschwenkbarer Sperrhaken 17 ein, und zwar nur so weit, daß zwischen dem vorderen Ende des Sperrhakens 17 und dem hinteren Rand des Betätigungsbügels 18 noch eine Distanz a vorhanden ist, innerhalb der das Führungsrohr 12 durch entsprechende Verschiebung des Betätigungsbügels 18 in Richtung des Pfeiles F gegen die Kraft der Rückstellfeder 19 verschoben werden kann.

Die Anordnung des Sperrhakens 17 ist derart, daß auch bei vollständiger Überwindung der Distanz a durch den Betätigungsbügel 18 das hintere Ende 42 der Rastzungen 24 noch nicht das vordere Ende 29 des Kulissenansatzes 33 berührt, jedoch diesem sehr nahe liegt.

Über das Führungsrohr 12 ist koaxial ein ebenfalls kreiszylindrisch ausgebildetes Außenrohr 13 geschoben, welches an seinem hinteren Endbereich einen ringförmigen Befestigungskörper 43 trägt, der an seinem hinteren Ende axial vorstehende Rastarme 27 aufweist, die das vordere Ende des rohrförmig ausgebildeten Handgriffs 14 axial übergreifen und in eine unmittelbar vor dem Anschlag 30 vorgesehene ringförmige Rastnut 28 von außen lösbar eingreifen.

Erfindungsgemäß sind die hinteren Enden 42 der Rastköpfe 24' konisch ausgebildet, während die vordere Stirnfläche des Kulissenansatzes 33 als zu den Enden 42 komplementäre Kulisse 29 ausgebildet ist.

Am hinteren Ende des Elektrodenrohrs 11 liegt eine Kontaktfeder 22 elektrisch leitend an, die an einem Isolierblock 44 befestigt und mit einem Hochspannungs-Zuleitungskabel 23 elektrisch leitend verbunden ist.

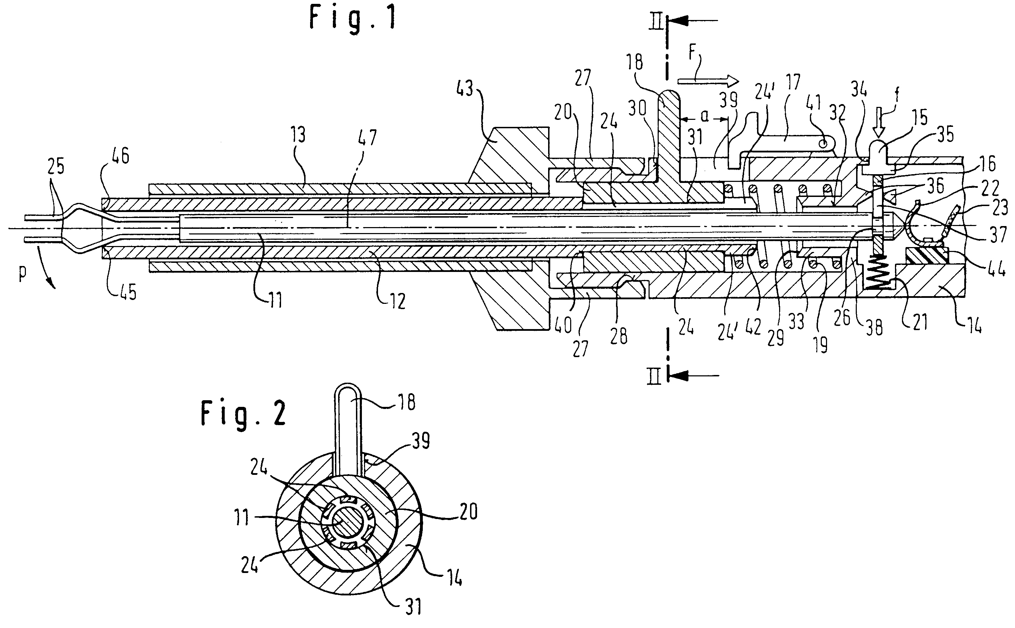

Für den Fall, daß - wie in Fig. 1 dargestellt - zwei gegeneinander isolierte Arbeitselektroden 25 vorgesehen sind, müssen am hinteren Ende des Elektrodenrohres 11 entsprechend zwei beabstandete Kontaktfedern 22 nebeneinander angeordnet sein und mit zwei Hochfrequenz-Spannungszuführungsleitungen verbunden werden.

Die Arbeitsweise des beschriebenen Hochfrequenz-Chirurgieinstrumentes ist wie folgt:

Im normalen Betrieb wird eine Hochfrequenzspannung an die beiden Elektroden 25 angelegt. Durch Verschieben des Bügels 18 in Richtung des Pfeiles F innerhalb der Distanz a kann das Führungsrohr 12 relativ zum Elektrodenrohr 11 nach hinten verschoben werden, wodurch die vorderen, diametral gegenüberliegenden Innenkanten 45, 46 des Führungsrohrs 12 auf den radial äußeren Flanken der beiden Arbeitselektroden 25 nach hinten gleiten, so daß sich die untere Elektrode 25 in Richtung des Pfeiles p und die obere in entgegengesetzter Richtung federnd voneinander wegspreizen. Beim anschließenden Wiedervorschieben der Führungshülse 20, was durch die Kraft der Feder 19 geschieht, wenn die in Richtung des Pfeiles F wirkende Verstellkraft verschwindet, gleiten die Kanten 45, 46 auf den Elektroden 25 nach vorn und bewegen diese federnd aufeinanderzu.

Durch das Hin- und Herverschieben des Bügels 18 kann somit eine zangenartige Bewegung der beiden Arbeitselektroden 25 relativ zueinander hervorgerufen werden.

Soll das Instrument zerlegt werden, so kann dies dadurch geschehen, daß zunächst auf den Druckknopf 15 in Richtung des Pfeiles f gedrückt wird, wodurch der Kupplungsriegel 16 außer Eingriff mit dem Elektrodenrohr 11 kommt. Dieses kann dann nach vorn vollständig aus dem Führungsrohr 12 herausgezogen werden.

Anschließend wird der Sperrhaken 17 um sein Gelenk 41 nach oben geschwenkt, wodurch auch der hintere Bereich des Radialschlitzes 39 für den Bügel 18 zugänglich wird. Dieser kann daher über die normale Betriebsdistanz a weiter nach hinten verschoben werden, wobei das hintere Ende 42 der Rastköpfe 24' mit der Kulisse 29 in Eingriff kommt.

Aufgrund der aus der Zeichnung ersichtlichen Schrägstellung der miteinander in Eingriff kommenden Flächen 42, 29 und der Tatsache, daß das Elektrodenrohr 11 bereits herausgezogen ist, weichen die Rastzungen 24 nunmehr radial nach innen aus und kommen dadurch außer axialen Eingriff mit der Führungshülse 20. Jetzt muß noch ein geringer axialer Spielraum innerhalb des Radialschlitzes 39 zur Verfügung stehen, damit nach dem Außer-Eingriff-Kommen der Rastköpfe 24' mit der Führungshülse 20 die Führungshülse 20 sich über den Außenumfang der Rastköpfe 24' schiebt.

Nunmehr ist der das Herausziehen des Führungsrohres 12 nach vorn verhinderte axiale Eingriff zwischen den Rastzungen 24 und der Führungshülse 20 beseitigt, so daß jetzt auch das Führungsrohr 12 nach vorn aus dem Außenrohr 13 herausgezogen werden kann.

Anschließend müssen nur noch die Rastarme 27 des Befestigungskörpers 43 aus der ringförmigen Rastnut 28 entfernt werden, was entweder durch ein Werkzeug oder durch eine entsprechend große axiale Abzugskraft herbeigefuhrt werden kann, sofern die vorderen Flanken der Rastnut 28 - wie in Fig. 1 dargestellt - etwas schräg ausgebildet sind.

Das Instrument ist jetzt vollständig zerlegt, und die vier erhaltenen Einzelteile, nämlich das Elektrodenrohr 11, das Führungsrohr 12, das Außenrohr 13 sowie der Handgriff 14 können einer intensiven Reinigung und gegebenenfalls Sterilisierung zugeführt werden.

### Bezugszeichenliste

- 11: Elektrodenrohr
- 12: Führungsrohr
- 13: Außenrohr
- 14: Betätigungshandgriff
- 15: Druckknopf
- 16: Kupplungsriegel
- 17: Sperrhaken
- 18: Betätigungsbügel
- 19: Rückstellfeder
- 20: Führungshülse
- 21: Riegelfeder
- 22: Kontaktfeder
- 23: Hochfrequenz-Spannungszuleitung
- 24: Rastzunge
- 24': Rastkopf
- 25: Arbeitselektrode
- 26: Rastnut
- 27: Rastarm
- 28: Rastnut
- 29: Kulisse
- 30: Anschlag
- 31: Bohrung
- 32: Bohrung
- 33: Kulissenansatz
- 34: Bohrung
- 35: Erweiterung
- 36: Querführung
- 37: Öffnung
- 38: Ringwand
- 39: Radialschlitz
- 40: Ringstufe
- 41: Gelenk
- 42: Ende
- 43: Befestigungskörper
- 44: Isolierblock
- 45: Innenkante
- 46: Rand
- 47: Achse

## Patentansprüche

1. Zerlegbares medizinisches Instrument, insbesondere Hochfrequenz-Chirurgieinstrument,
mit einem zentralen Arbeitsstab (11), an dessen vorderem Ende zumindest ein Arbeitswerkzeug (25) vorgesehen ist,
mit einem den Arbeitsstab (11) umgebenden Führungsrohr (12), das zur Betätigung des zumindest einen Arbeitswerkzeugs (25) relativ zum Arbeitsstab (11) axial in einem bestimmten Distanzbereich verschiebbar ist, und
mit einem den Arbeitsstab (11) und das Führungsrohr (12) aufnehmenden Handgriff (14),
wobei der Handgriff (14), der Arbeitsstab (11) und das Führungsrohr (12) voneinander trennbar ausgebildet sind,
dadurch **gekennzeichnet,**
daß die hinteren Enden des Arbeitsstabes (11) und des Führungsrohrs (12) innerhalb des Handgriffs (14) angeordnet sind,
daß der Arbeitsstab (11) lösbar innerhalb des Handgriffs (14) befestigt ist,
daß der hintere Endbereich des Führungsrohrs (12) im Bereich vor der Stelle, wo der Arbeitsstab (11) lösbar befestigt ist, in einer am Handgriff (14) bewegbar gelagerten Führungshülse (20) angeordnet und mit dieser lösbar verbunden ist und
daß die Verbindung zwischen dem Führungsrohr (12) und der Führungshülse (20) von außen lösbar ist.

2. Instrument nach Anspruch 1,
dadurch gekennzeichnet,
daß der zentrale Arbeitsstab (11) als Elektrodenrohr ausgebildet ist.

3. Instrument nach Anspruch 2,
dadurch gekennzeichnet,
daß zumindest ein Arbeitswerkzeug (25) als Arbeitselektrode ausgebildet ist.

4. Instrument nach einem der Ansprüche 1 bis 3,
dadurch gekennzeichnet,
daß an dem hinteren Ende des zentralen Arbeitsstabs (11) Anschlüsse zur Versorgung des Arbeitswerkzeugs (25), insbesondere ein bzw. mehrere Hochfrequenz-Spannungsanschlüsse (22) vorgesehen sind.

5. Instrument nach einem der vorhergehenden Ansprüche,
dadurch gekennzeichnet,
daß das Führungsrohr (12) den Arbeitsstab (11) koaxial umgibt.

6. Instrument nach einem der vorhergehenden Ansprüche,
dadurch gekennzeichnet,
daß ein das Führungsrohr (12) vorzugsweise koaxial umgebendes Außenrohr (13) vorgesehen ist.

7. Instrument nach Anspruch 6,
dadurch gekennzeichnet,
daß das Außenrohr (13) von dem Handgriff (14) aufgenommen ist.

8. Instrument nach Anspruch 6 oder 7,
dadurch gekennzeichnet,
daß das Außenrohr (13) von dem Handgriff (14), dem Arbeitsstab (11) und/oder dem Führungsrohr (12) trennbar ausgebildet ist.

9. Instrument nach einem der vorhergehenden Ansprüche,
dadurch gekennzeichnet,
daß der Arbeitsstab (11), das Führungsrohr (12) und gegebenenfalls das Außenrohr (13) axial voneinander und vom Handgriff (14) abziehbar sind.

10. Instrument nach einem der vorhergehenden Ansprüche,
dadurch gekennzeichnet,
daß der Arbeitsstab (11), das Führungsrohr (12) und gegebenenfalls das Außenrohr (13) mit dem Handgriff (14) durch Rast- und/oder Riegel- und/oder Schnappverbindungen (16, 26; 20, 24; 27, 28) lösbar verbunden sind.

11. Instrument nach einem der vorhergehenden Ansprüche,
dadurch gekennzeichnet,
daß der Arbeitsstab (11), das Führungsrohr (12) und gegebenenfalls das Außenrohr (13) axial auf und/oder in einen rohrartig ausgebildeten Handgriff (14) einsetzbar sind.

12. Instrument nach einem der vorhergehenden Ansprüche,
dadurch gekennzeichnet,
daß der Arbeitsstab (11) an seinem im Handgriff (14) befindlichen hinteren Endbereich wenigstens eine radiale Vertiefung, insbesondere eine Ringnut (26) aufweist, in die ein am Handgriff (14) querverschiebbar gelagerter und von außen betätigbarer Kupplungsriegel (16) lösbar eingreift, wobei vorzugsweise der Eingriff derart ist,
daß die zunächst vorhandene Drehbarkeit des Arbeitsstabes (11) um seine Achse aufgehoben ist.

13. Instrument nach einem der vorhergehenden Ansprüche,
dadurch gekennzeichnet,
daß die Führungshülse (20) am Handgriff (14) axial verschiebbar und/oder drehbar gelagerten ist.

14. Instrument nach einem der vorhergehenden Ansprüche,
dadurch gekennzeichnet,
daß die Verbindung zwischen dem Führungsrohr (12) und der Führungshülse (20) von außen durch axiale Verschiebung und/oder Verdrehung der Führungshülse (20) lösbar ist.

15. Instrument nach einem der vorhergehenden Ansprüche,
dadurch gekennzeichnet,
daß die Führungshülse (20) axial von mit ihr in axialem Eingriff stehenden Rastzungen (24) am Ende des Führungsrohres (12) durchgriffen ist, die durch Axialverschiebung der Führungshülse (20) nach hinten gegen eine handgriffeste Kulisse (29) radial nach innen verschiebbar und damit außer Eingriff mit der Führungshülse (20) bringbar sind.

16. Instrument nach einem der vorhergehenden Ansprüche,
dadurch gekennzeichnet,
daß die Führungshülse (20) federnd nach vorn gegen einen Anschlag (30) vorgespannt ist.

17. Instrument nach einem der Ansprüche 13 bis 16,
dadurch gekennzeichnet,
daß ein erster axialer Verschiebebereich (a) für die Führungshülse (20) und das Führungsrohr (12) vorgesehen ist, der einer axialen Relativverschiebung zwischen dem Arbeitsstab (11) und dem Führungsrohr (12) dient, und
daß ein zweiter Verschiebebereich sich nach hinten anschließt, der der Lösung der Verbindung zwischen dem Führungsrohr (12) und der Führungshülse (20) dient.

18. Instrument nach Anspruch 17,
dadurch gekennzeichnet,
daß am Ende des ersten Verschiebebereiches (a) eine von außen lösbare Sperre (17) vorgesehen ist, und daß die Führungshülse (20) den zweiten Verschiebebereich erst nach Entfernen der Sperre (17) durchlaufen kann.

19. Instrument nach einem der Ansprüche 6 bis 18,
dadurch gekennzeichnet,
daß das Außenrohr (13) auf das vordere Ende des Handgriffes (14) lösbar aufschiebbar und dort festlegbar ist.

20. Instrument nach einem der vorhergehenden Ansprüche,
dadurch gekennzeichnet,
daß das bzw. die Arbeitswerkzeuge, insbesondere Arbeitselektroden (25) federnd voneinander wegspreizbar vorgespannt und durch die vordere Innenkante (45, 46) des axial verschiebbaren Führungsrohres (12) je nach der axialen Position des Führungsrohres (12) mehr oder weniger gegen die Vorspannung zusammengedrückt werden.

21. Instrument nach einem der vorhergehenden Ansprüche,
dadurch gekennzeichnet,
daß der Arbeitsstab (11) axial hohl ausgebildet ist und
daß durch den axialen Hohlraum Versorgungsleitungen für die Arbeitswerkzeuge (25) verlegt sind.

## Claims

1. Dismantlable medical instrument, in particular a high-frequency surgical instrument comprising a central working rod (11), at the front end of which there is provided at least one working tool (25), a guide tube (12) surrounding the working rod (11), the guide tube (12) being axially displaceable within a certain range of distance relative to the working rod (11) for the actuation of the at least one working tool (25) and a handle (14) receiving the working rod (11) and the guide tube (12), with the handle (14), the working rod (11) and the guide tube (12) being made separable from one another, characterized in that the rear ends of the working rod (11) and of the guide tube (12) are arranged within the handle (14), in that the working rod (11) is releasably secured inside the handle (14), in that, in the region ahead of the position where the working rod (11) is releasably secured, the rear end region of the guide tube (12) is arranged in a guide sleeve (20) movably mounted at the handle (14) and is releasably connected to the guide sleeve (20); and in that the connection between the guide tube (12) and the guide sleeve (20) can be released from the outside.

2. Instrument in accordance with claim 1, characterized in that the central working rod (11) is formed as an electrode tube.

3. Instrument in accordance with claim 2, characterized in that at least one working tool (25) is formed as a working electrode.

4. Instrument in accordance with one of the claims 1 to 3, characterized in that connections for the supply of the working tool (25), in particular one or more high-frequency voltage connections (22), are provided at the rear end of the central working rod (11).

5. Instrument in accordance with one of the preceding claims, characterized in that the guide tube (12) coaxially surrounds the working rod (11).

6. Instrument in accordance with one of the preceding claims, characterized in that an outer tube (13) is provided which preferably coaxially surrounds the guide tube (12).

7. Instrument in accordance with claim 6, characterized in that the outer tube (13) is received by the handle (14).

8. Instrument in accordance with claim 6 or 7, characterized in that the outer tube (13) is designed to be separable from the handle (14), from the working rod (11) and/or from the guide tube (12).

9. Instrument in accordance with one of the preceding claims, characterized in that the working rod (11), the guide tube (12), and optionally the outer tube (13) can be drawn off axially from one another and from the handle (14).

10. Instrument in accordance with one of the preceding claims, characterized in that the working rod (11), the guide tube (12) and optionally the outer tube (13) with the handle (14) are releasably connected by latch connections and/or lock connections and/or snap connections (16, 26; 20, 24; 27, 28).

11. Instrument in accordance with one of the preceding claims, characterized in that the working rod (11), the guide tube (12) and optionally the outer tube (13) can be axially inserted onto and/or into a handle (14) of tubular design.

12. Instrument in accordance with one of the preceding claims, characterized in that the working rod (11) has at least one radial recess, in particular a ring groove (26), at its rear end region located in the handle (14), with a coupling lock member (16), which is transversely, displaceably journalled at the handle (14), can be actuated from the outside engaging into the recess, with the engagement preferably being such that the existing rotatability of the working rod (11) about its axis is cancelled.

13. Instrument in accordance with one of the preceding claims, characterized in that the guide sleeve (20) is axially displaceably and/or rotatably mounted at the handle (14).

14. Instrument in accordance with one of the preceding claims, characterized in that the connection between the guide tube (12) and the guide sleeve (20) can be released from the outside by axial displacement and/or rotation of the guide sleeve (20).

15. Instrument in accordance with one of the preceding claims, characterized in that latch tongues (24) at the end of the guide tube (20) and standing in axial engagement with the guide tube (20) pass axially through the guide tube (20) and can be displaced radially inwardly and thus brought out of engagement with the guide sleeve (20) by axial displacement of the guide sleeve (20) to the rear against a cam track feed relative to the handle (14).

16. Instrument in accordance with one of the preceding claims, characterized in that the guide sleeve (20) is biased resiliently forwardly against an abutment (30).

17. Instrument in accordance with one of the claims 13 to 16, characterized in that a first axial displacement region (a) is provided for the guide sleeve (20) and the guide tube (12) and serves for a relative axial displacement between the working rod (11) and the guide tube (12), and in that a second displacement region adjoins it to the rear, which serves for the release of the connection between the guide tube (12) and the guide sleeve (20).

18. Instrument in accordance with claim 17, characterized in that a catch (17) releasable from the outside is provided at the end of the first displacement region (a), and in that the guide sleeve (20) can first pass through the second displacement range after removal of the catch (17).

19. Instrument in accordance with one of the claims 6 to 18, characterized in that the outer tube (13) can be releasably pushed onto the front end of the handle (14) and can be fixed there.

20. Instrument in accordance with one of the preceding claims, characterized in that the working tool or the working tools, in particular working electrodes (25) are resiliently biased to be able to spread away from each other and are pressed together to a greater or lesser degree against the bias by the front inner edge (45, 46) of the axially displaceable guide tube (12) depending on the axial portion of the guide tube (12).

21. Instrument in accordance with one of the preceding claims, characterized in that the working rod (11) is designed axially hollow and in that supply lines for the working tools (25) are provided through the axial hollow cavity.

## Revendications

1. Instrument médical démontable - en particulier instrument de chirurgie à haute fréquence - démontable, comportant une barre de travail centrale (11), à l'extrémité avant de laquelle il est prévu au moins un outil de travail (25), comportant un tube de guidage (12), entourant la barre de travail (11) et pouvant être déplacé axialement sur une certaine distance par rapport à la barre de travail (11) pour actionner au moins un des outils de travail (25), et comportant une poignée (14) recevant la barre de travail (11) et le tube de guidage (12), cependant que la poignée (14), la barre de travail (11) et le tube de guidage (12) peuvent être séparés les uns des autres, caractérisé en ce que les extrémité arrière de la barre de travail (11) et du tube de guidage (12) sont disposées à l'intérieur de la poignée (14), que la barre de travail (11) est fixée de manière amovible à l'intérieur de la poignée (14), que, dans la zone devant l'endroit où la barre de travail (11) est fixée de manière amovible, la zone d'extrémité arrière du tube de guidage (12) est disposée dans un fourreau de guidage (20) logé de manière mobile sur la poignée (14), et est reliée de manière amovible au fourreau de guidage et que la liaison entre le tube de guidage (12) et le fourreau de guidage (20) peut être supprimée de l'extérieur.

2. Instrument suivant la revendication 1, caractérisé en ce que la barre de travail centrale (11) est constituée par un tube à électrodes.

3. Instrument suivant la revendication 2, caractérisé en ce qu'au moins un outil de travail (25) est constitué par une électrode de travail.

4. Instrument suivant une des revendications 1 à 3, caractérisé en ce qu'à l'extrémité arrière de la barre de travail centrale (11), il est prévu des connexions pour l'alimentation de l'outil de travail (25), en particulier une ou plusieurs connexions électriques (22) à haute fréquence.

5. Instrument suivant l'une des revendications précédentes, caractérisé en ce que le tube de guidage (12) entoure coaxialement la barre de travail (11).

6. Instrument suivant une des revendications précédentes, caractérisé en ce qu'il est prévu un tube extérieur (13) entourant avantageusement d'une manière coaxiale le tube de guidage (12).

7. Instrument suivant la revendication 6, caractérisé en ce que le tube extérieur (13) est logé dans la poignée (14).

8. Instrument suivant l'une des revendication 6 et 7, caractérisé en ce que le tube extérieur (13) peut être séparé de la poignée (14), de la barre de travail (11) et/ou du tube de guidage (12).

9. Instrument suivant l'une des revendications précédentes, caractérisé en ce que la barre de travail (11), le tube de guidage (12) et, le cas échéant, le tube extérieur (13) peuvent être retirés axialement les uns des autres ainsi que de la poignée (14).

10. Instrument suivant l'une des revendications précédentes, caractérisé en ce que la barre de travail (11), le tube de guidage (12) et, le cas échéant, le tube extérieur (13) sont reliés de manière amovible à la poignée (14) par des éléments d'assemblage à encrantement et/ou à verrouillage et/ou à encliquetage (16, 26; 20, 24; 27, 28).

11. Instrument suivant l'une des revendications précédentes, caractérisé en ce que la barre de travail (11), le tube de guidage (12) et, le cas échéant, le tube extérieur (13) sont à insérer axialement sur et/ou dans une poignée tubulaire (14).

12. Instrument suivant l'une des revendications précédentes, caractérisé en ce que la barre de travail (11) présente, dans sa zone d'extrémité arrière se trouvant dans la poignée (14), au moins une cavité radiale, en particulier une rainure annulaire (26), dans laquelle un verrou d'accouplement (16), mobile transversalement sur la poignée (14) et pouvant être actionné de l'extérieur, pénètre de manière amovible, cependant que la pénétration est conçue avantageusement de sorte que la capacité de rotation - existant d'abord - de la barre de travail (11) autour de son axe soit supprimée.

13. Instrument suivant l'une des revendications précédentes, caractérisé en ce que le fourreau de guidage (20) est logé mobile axialement linéairement et/ou en rotation sur la poignée (14).

14. Instrument suivant l'une des revendications précédentes, caractérisé en ce que la liaison entre le tube de guidage (12) et le fourreau de guidage (20) est amovible de l'extérieur par déplacement axial et/ou rotation du fourreau de guidage (20).

15. Instrument suivant l'une des revendications précédentes, caractérisé en ce que le fourreau de guidage (20) est pénétré axialement, à l'extrémité du tube de guidage (12), par des languettes d'encrantement (24) en prise axiale avec lui, qui, par déplacement axial du fourreau de guidage (20) vers l'arrière, sont mobiles radialement vers l'intérieur contre une coulisse fixe (29) de la poignée, de sorte qu'elle peuvent être amenées hors de prise par rapport au fourreau de guidage (20).

16. Instrument suivant l'une des revendications précédentes, caractérisé en ce que le fourreau de guidage (20) est précontraint élastiquement vers l'avant, contre l'action d'une butée (30).

17. Instrument suivant l'une des revendications 13 à 16, caractérisé en ce qu'une première zone axiale (a) de déplacement est prévue pour le fourreau de guidage (20) et le tube de guidage (12), qui sert au déplacement relatif axial entre la barre de travail (11) et le tube de guidage (12), et qu'une deuxième zone de déplacement se raccorde vers l'arrière, cette zone étant utilisée pour annuler la liaison entre le tube de guidage (12) et le fourreau de guidage (20).

18. Instrument suivant la revendication 17, caractérisé en ce qu'à l'extrémité de la première zone (a) de déplacement, il est prévu un blocage (17) amovible de l'extérieur et en ce que le fourreau de guidage (20) ne peut passer la deuxième zone de déplacement qu'une fois enlevé le blocage (17).

19. Instrument suivant l'une des revendications 6 à 18, caractérisé en ce que le tube extérieur (13) peut être poussé de manière amovible sur l'extrémité avant de la poignée 14 et qu'il peut y être fixé.

20. Instrument suivant l'une des revendications précédentes, caractérisé en ce que le ou les outils de travail, en particulier les électrodes de travail (25), peuvent être précontraintes et écartés élastiquement l'un par rapport à l'autre et peuvent, sous l'action des bords intérieurs avant (45, 46) du tube de guidage (12) mobile axialement et suivant la position axiale du tube de guidage (12), être comprimés plus ou moins contre la précontrainte.

21. Instrument suivant une des revendications précédentes, caractérisé en ce que la barre de travail (11) est axialement creuse et en ce que dans l'espace creux axial sont posées des conduites d'alimentation pour les outils de travail (25).
